Europäisches Patentamt

European Patent Office

Office européen des brevets

(11) Publication number: **0 261 771 B1**

# EUROPEAN PATENT SPECIFICATION

(45) Date of publication of patent specification: **01.04.92** (51) Int. Cl.⁵: **C07C 29/15**, C07C 31/04

(21) Application number: **87306437.2**

(22) Date of filing: **21.07.87**

(54) **Manufacture of organic liquids.**

(30) Priority: **23.09.86 US 910640**

(43) Date of publication of application:
**30.03.88 Bulletin 88/13**

(45) Publication of the grant of the patent:
**01.04.92 Bulletin 92/14**

(84) Designated Contracting States:
**DE ES FR GB IT NL SE**

(56) References cited:
**FR-A- 1 046 822**
**GB-A- 2 047 249**
**US-A- 4 628 066**

(73) Proprietor: **FOSTER WHEELER ENERGY LIMITED**
**Foster Wheeler House Station Road**
**Reading Berkshire, RG1 1LX(GB)**

(72) Inventor: **Kowal, Wieslaw Mark**
**Sunnyview Binfield Heath**
**Henley Oxfordshire(GB)**
Inventor: **Skinner, Geoffrey Frederick**
**Kimblewick Sutherland Avenue**
**Reading Berkshire(GB)**

(74) Representative: **Bowman, Paul Alan et al**
**LLOYD WISE, TREGEAR & CO. Norman**
**House 105-109 Strand**
**London WC2R OAE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to the manufacture of organic liquids. In particular this invention relates to the manufacture of organic liquids selected from methanol, ethanol and higher alcohols, Fischer-Tropsch products and olefins from feed gases containing hydrogen, nitrogen, and carbon monoxide produced by the partial oxidation of hydrocarbons and other carbonaceous materials in air.

Methods of producing organic liquids from mixtures of hydrogen and carbon monoxide (commonly called synthesis gas) are well known in the art. Methanol, for instance, is normally manufactured by reacting carbon oxides (mainly carbon monoxide) with hydrogen over a copper containing catalyst, whereby the carbon oxides and hydrogen are converted to methanol.

Another well-known process that uses synthesis gas is the Fischer-Tropsch system, which is used to manufacture synthetic transportation fuels (gasoline and diesel).

Higher alcohols and olefins are among other products that may be manufactured from synthesis gas.

To achieve a more complete conversion of the synthesis gas into products, the effluent gas from the organic synthesis reactor is usually recycled to the reactor inlet, thus forming the well-known 'synthesis loop'. Examples of processes incorporating such a synthesis loop are disclosed in British Patent Publication No. 2047249 and French Patent Specification No. 1046822. If the synthesis gas used in such processes is produced by the partial combustion of a hydrocarbon or other carbonaceous material with air, the nitrogen present in the air will build-up to undesirable levels in the synthesis loop.

In order to synthesize organic liquids without an undesirable build-up of nitrogen in the synthesis loop, the nitrogen has to either be removed from the synthesis gas or the partial combustion process has to be accomplished in the absence of air, i.e., with oxygen.

The present invention seeks to provide a process for synthesizing certain organic liquids, such as methanol, from gases derived from the partial combustion of hydrocarbons or other carbonaceous materials with air without the undesirable build-up of nitrogen occurring within the system.

According to the present invention there is provided a process for the synthesis of organic liquids selected from methanol, ethanol and higher alcohols, Fischer-Tropsch products and olefins from a feed gas containing hydrogen, nitrogen and carbon monoxide produced by the partial oxidation of hydrocarbons and other carbonaceous materials with air, which process comprises:

(a) passing the feed gas through a series of two or more synthesis reactors; and
(b) removing the organic liquid from each reactors.

The feed gas may be produced by the partial combustion of hydrocarbons, such as natural gas or petroleum, or other carbonaceous material, such as coal or coke, with air. Steam can also be present during the combustion process.

Any process can be used for the partial combustion. An example of a process for the partial combustion of hydrocarbons is the Texaco partial oxidation process which operates at around 1371°C (2500°F) and between $1.01 \times 10^6$ and $1.01 \times 10^7$ $Nm^{-2}$ (10 and 100 atmospheres). An example of a process for the partial combustion (i.e., gasification) of coal is the U-gas fluid bed process, which operates at around 982°C (1800°F) and at $5.05 \times 10^5$ and $1.52 \times 10^6$ $Nm^{-2}$ (5 to 15 atmospheres).

Whichever process is used for the partial combustion operation, the synthesis gas produced will have as its main constituents hydrogen, carbon monoxide and nitrogen.

For the efficient operation of the organic synthesis, it may be necessary for the synthesis gas to be essentially free of sulfur compounds, carbon dioxide and/or water vapor.

Sulfur compounds, if present in the feedstock, can be removed either from the feedstock before partial combustion or afterwards. In the case of a hydrocarbon gas feedstock containing a few parts per million of sulfur compounds, this would be best accomplished upstream of the partial combustion using an absorbent, such as zinc oxide, operating at around 371°C (700°F). In the case of a hydrocarbon gas with a large sulfur content (over 500 volume parts per million (vpm), or a coal, coke, or petroleum oil, the removal of sulfur is best accomplished by washing the partial combustion product gas with a solvent, such as monoethanolamine solution at 37.8°C (100°F).

Carbon dioxide can be removed from the partial combustion product gas by a similar monoethanolamine process or other established washing process. Water vapor can be removed from the partial combustion product gas by cooling and condensation.

The resulting purified gas stream comprising mainly hydrogen, carbon monoxide and nitrogen is passed through a series of two or more reactors wherein the organic synthesis desired is performed.

Any number of synthesis reactors can be used within the scope of this invention. The number of reactors is used in the organic liquid synthesis generally ranges from two to four, depending on the liquid produced.

Any catalyst known in the prior art for synthesizing the organic liquids can be used in this invention. Typical catalysts include copper and zinc-containing catalysts for methanol manufacture and iron oxide-based catalysts for Fischer-Tropsch processing.

The organic liquid synthesis reactors generally operate at temperatures and pressures suitable to the catalyst being used. Typically, the synthesis reactor will operate at temperatures ranging from 93.3 to 260°C (200 to 500°F) and pressures ranging from $1.01 \times 10^5$ to $1.01 \times 10^7$ $Nm^{-2}$ (atmospheric to 100 atmospheres).

The organic product is removed from the effluent stream from each reactor. This separation is generally carried out by cooling and condensing the organic product from the effluent. The condensation process takes place at a temperature ranging from -128.9 to 148.9°C (-200°F to 300°F) and a pressure ranging from $1.01 \times 10^5$ to $1.01 \times 10^7$ $Nm^{-2}$ (atmospheric pressure to 100 atmospheres).

Cooling and condensing can be supplemented or replaced, if desirable, by another separation process, such as washing with a suitable liquid or adsorption on a solid such as with molecular sieves.

The reactor effluent gas, after product separation, is then passed to the next reactor inlet.

The gas leaving the product separator following the final reactor is a waste gas stream that can be expanded to low pressure for use as a fuel. Optionally the waste gas stream can be processed to recover its hydrogen and carbon monoxide content for recycle to a point upstream.

Examples of such recovery include:

(1) Adding steam to the waste gas stream, passing it over to a CO shift catalyst, such as iron oxide, so that the steam oxidizes most of the carbon monoxide to carbon dioxide with release of an equal volume of additional hydrogen, washing out the carbon dioxide present with a solvent such as monoethanolamine, and finally condensing out most of the nitrogen content of the gas to leave a gas stream consisting mainly of hydrogen. This may then be recycled to the synthesis operation.

(2) Passing the waste gas through an adsorbent, such as a molecular sieve, so as to substantially separate its hydrogen content so that the hydrogen may be recycled to the synthesis operation.

(3) Washing carbon monoxide from the waste gas using a solvent such as "COSORB" such that the carbon monoxide removed may be recycled to the synthesis operation.

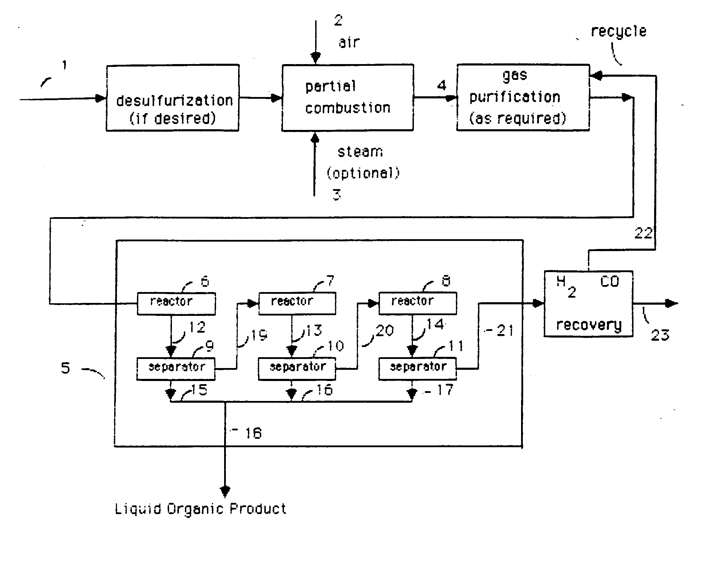

The invention will now be described by way of example with reference to the accompanying drawing, in which:

the figure is a flow diagram of the preferred embodiment of the present invention.

Referring to the figure, in a preferred embodiment of this invention, hydrocarbons or other carbonaceous materials are fed via line 1 to an optional desulfurization step and then to a partial combustion step, where the hydrocarbons or other carbonaceous materials are combined with air via line 2 and optionally steam line 3 to partially combust the hydrocarbons or other carbonaceous materials to form a synthesis gas.

The synthesis gas leaves the partial combustion steam via line 4 (where it may be subject to an optional purification step) and is then passed to an organic liquid synthesis zone 5. In the organic liquid synthesis zone, the synthesis gas is passed through a series of reactors, 6, 7 and 8 which convert the synthesis gas to the desired fluid product. After passing through each reactor, the unreacted synthesis gas and organic liquid are fed to separators 9, 10 and 11 via lines 12, 13 and 14 for separation of the organic liquid from the untreated synthesis gas. The organic liquid is then recovered via lines 15, 16, 17 and 18. The unreacted synthesis gas is passed to the next reactor via line 19 and 20. After a final separation step, a waste gas is recovered via line 21. The waste gas can be treated to recover $H_2$ and CO and recycled via line 22 or it can be passed via line 23 for further use, such as a fuel.

COSORB is a trade name.

## Claims

1. A process for the synthesis of organic liquids selected from methanol, ethanol and higher alcohols, Fischer-Tropsch products and olefins from a feed gas containing hydrogen, nitrogen and carbon monoxide produced by the partial oxidation of hydrocarbons and other carbonaceous materials with air, which process comprises:

   (a) passing the feed gas through a series of two or more synthesis reactors; and

   (b) removing the organic liquid from each reactor.

2. A process as claimed in Claim 1 in which the organic liquid is methanol.

3. A process as claimed in Claim 1 or Claim 2, where two to four organic liquid synthesis reactors are present.

4. A process as claimed in any one of Claims 1 to 3 in which waste gas from the final synthesis reactor is expanded to low pressure for use as a fuel.

5. A process as claimed in any preceding Claim in which waste gas from the final synthesis reactor is treated to recover the hydrogen or carbon monoxide content for recycle to an earlier stage in the process.

6. A process as claimed in any preceding Claim in which the synthesis reactors operate at a temperature ranging from 93.3 to 260°C (200 to 500°F) and a pressure ranging from $1.01 \times 10^5$ to $1.01 \times 10^7$ Nm$^{-2}$ (atmospheric pressure to 100 atmospheres) in the presence of a catalyst comprising copper, iron and zinc compounds as the principal active constituents.

7. A process as claimed in any preceding Claim in which the effluent stream from each reactor is cooled to condense and separate the organic liquid therefrom.

8. A process as claimed in Claim 7 in which the condensation process takes place at a temperature ranging from -128.9 to 148.9°C (-200 to 300°F) and a pressure ranging from $1.01 \times 10^5$ to $1.01 \times 10^7$ Nm$^{-2}$ (atmospheric pressure to 100 atmospheres).

**Revendications**

1. Procédé de synthèse de liquides organiques choisis parmi le méthanol, l'éthanol et les alcools supérieurs, les produits de Fischer-Tropsch et les oléfines à partir d'un gaz d'alimentation contenant de l'hydrogène, de l'azote et du monoxyde de carbone, obtenu par l'oxydation partielle d'hydrocarbures et autres matières carbonées avec l'air, lequel procédé comprend les opérations consistant à :
    (a) faire passer le gaz d'alimentation à travers une série de deux réacteurs de synthèse ou davantage ; et
    (b) retirer le liquide organique à partir de chaque réacteur.

2. Procédé selon la revendication 1, dans lequel le liquide organique est le méthanol.

3. Procédé selon la revendication 1 ou la revendication 2, où deux à quatre réacteurs de synthèse de liquide organique sont présents.

4. Procédé selon l'une quelconque des revendications 1 à 3, dans lequel du gaz résiduaire provenant du réacteur de synthèse final est détendu à basse pression pour être utilisé comme combustible.

5. Procédé selon l'une quelconque des revendications précédentes, dans lequel le gaz résiduaire provenant du réacteur de synthèse final est traité pour récupérer la teneur en hydrogène ou en monoxyde de carbone en vue d'un recyclage à un stade antérieur du procédé.

6. Procédé selon l'une quelconque des revendications précédentes, dans lequel les réacteurs de synthèse fonctionnent à une température se situant dans la plage de 93,3 à 260°C (200 à 500°F) et sous une pression se situant dans la plage de $1,01 \times 10^5$ à $1,01 \times 10^7$ Nm$^{-2}$ (pression atmosphérique à 100 atmosphères), en présence d'un catalyseur comprenant des composés du cuivre, du fer et du zinc, en tant que constituants actifs principaux.

7. Procédé selon l'une quelconque des revendications précédentes, dans lequel le courant d'effluent de chaque réacteur est refroidi pour en condenser et en séparer le liquide organique.

8. Procédé selon la revendication 7, dans lequel le procédé de condensation se déroule à une température se situant dans la plage de -128,9 à 148,9°C (-200 à 300°F) et sous une pression se situant dans la plage de $1,01 \times 10^5$ à $1,01 \times 10$ Nm$^{-2}$ (pression atmosphérique à 100 atmosphères).

**Patentansprüche**

1. Verfahren für die Synthese von organischen, aus Methanol, Ethanol und höheren Alkoholen, Fisher-Tropsch Produkten und Olefinen ausgewählten Flüssigkeiten aus einem Zufuhrgas, das Wasserstoff, Stickstoff und Kohlenmonoxid enthält, die durch die teilweise Oxidation von Kohlenwasserstoffen und anderen kohlenstoffhaltigen Materialien mit Luft erzeugt worden sind, welches Verfahren umfaßt:
    (a) das Leiten des Zufuhrgases durch eine Folge von zwei oder mehr Synthesereaktoren; und
    (b) die Abführung der organischen Flüssigkeit aus jedem Reaktor.

2. Verfahren nach Anspruch 1, in welchem die organische Flüssigkeit Methanol ist.

3. Verfahren nach Anspruch 1 oder Anspruch 2, worin zwei bis vier Synthesereaktoren für organische Flüssigkeit vorhanden sind.

4. Verfahren nach einem der Ansprüche 1 bis 3, in welchem Abgas aus dem letzten Synthesereaktor zur Verwendung als Brennstoff auf einen geringen Druck expandiert wird.

5. Verfahren nach einem vorhergehenden Anspruch, in welchem Abgas aus dem letzten Synthesereaktor zur Rückgewinnung des Wasserstoff- oder Kohlenmonoxidgehalts für die Rückführung zu einer früheren Stufe im Verfahren behandelt wird.

6. Verfahren nach einem vorhergehenden Anspruch, in welchem die Synthesereaktoren auf einer Temperatur, die von 93,3 bis 260 °C (200 bis 500 °F) reicht, und einem Druck, der von $1,01 \times 10^5$ bis $1,01 \times 10^7$ $Nm^{-2}$ (Atmosphärendruck bis zu 100 Atmosphären) reicht, in Anwesenheit eines Katalysators arbeiten, der Kupfer-, Eisen- und Zinkverbindungen als die aktiven Hauptbestandteile umfaßt.

7. Verfahren nach einem vorhergehenden Anspruch, in dem die Ausströmung aus jedem Reaktor zur Kondensierung und zur Abtrennung der organischen Flüssigkeit von ihr gekühlt wird.

8. Verfahren nach Anspruch 7, in welchem der Kondensationsprozeß auf einer Temperatur, die von -128,9 bis 148,9 °C (-200 bis 300 °F) reicht, und einem Druck, der von $1,01 \times 10^5$ bis $1,01 \times 10^7$ $Nm^{-2}$ (Atmosphärendruck bis zu 100 Atmosphären) reicht, abläuft.

2
air

1

desulfurization
(if desired)

partial
combustion

4

gas
purification
(as required)

recycle

steam
(optional)

3

22

6          7          8

reactor    reactor    reactor

12         13         14

19    20        -21

9          10         11

separator  separator  separator

15    -16        -17

5

-18

H₂  CO

recovery

23

Liquid Organic Product